Europäisches Patentamt

**European Patent Office**

Office européen des brevets

(19)

(11) Numéro de publication: **0 307 616 B1**

(12) **FASCICULE DE BREVET EUROPEEN**

(45) Date de publication de fascicule du brevet: **25.03.92**

(51) Int. Cl.⁵: **A61K 33/14**, //(A61K33/14, 33:04)

(21) Numéro de dépôt: **88112933.2**

(22) Date de dépôt: **09.08.88**

(54) **Médicament et produit vétérinaire pour le traitement de la stérilité.**

(30) Priorité: **26.08.87 FR 8711955**

(43) Date de publication de la demande:
**22.03.89 Bulletin 89/12**

(45) Mention de la délivrance du brevet:
**25.03.92 Bulletin 92/13**

(84) Etats contractants désignés:
**AT BE CH DE FR GB IT LI LU NL SE**

(56) Documents cités:
**US-A- 4 540 577**

(73) Titulaire: **Letourneur, Bernard**
**Route de Oizé Le Champ du Bourray Guécélard**
**F-72230 Arnage(FR)**

(72) Inventeur: **Letourneur, Bernard**
**Route de Oizé Le Champ du Bourray Guécélard**
**F-72230 Arnage(FR)**

(74) Mandataire: **Casalonga, Axel et al**
**BUREAU D.A. CASALONGA - JOSSE Morassistrasse 8**
**W-8000 München 5(DE)**

EP 0 307 616 B1

# EP 0 307 616 B1

**Description**

L'invention a pour objet un médicament pour le traitement de la stérilité chez l'homme et l'animal.

De nombreux couples sont stériles. La cause de la stérilité peut se trouver chez la femme ou chez l'homme.

La stérilité féminine peut être due à une malformation des organes sexuels, par exemple trompe bouchée ou à des maladies dues à des microorganismes, notamment des bactéries, virus et champignons.

La stérilité masculine est le plus souvent due à une insuffisance des spermatozoïdes en nombre et/ou en mobilité.

L'étude des spermatozoïdes sous forme de spermogramme comporte le dénombrement des spermatozoïdes, l'étude morphologique et l'évaluation de la proportion des spermatozoïdes normaux ainsi que l'étude de la mobilité, en précisant la proportion des spermatozoïdes mobiles pendant plusieurs heures à partir de leur prélèvement.

Le nombre des spermatozoïdes est exprimé par millilitre (ml) de sperme, d'une part, et dans la totalité de l'éjaculat, d'autre part.

Le volume de l'éjaculat est normalement de 2 à 5 ml.

On exprime la mobilité en dénombrant les formes à mobilité normale, les formes à mobilité diminuée et les formes immobiles, le nombre de ces différentes formes étant exprimé en pourcent par rapport au nombre total des spermatozoïdes.

On considère que le nombre des spermatozoïdes ne doit pas être inférieur à 60.000/ml. Habituellement, ce chiffre est de l'ordre du million.

En outre, il faut pour procréer qu'au moins 40% et de préférence 60% des spermatozoïdes soient à mobilité normale.

Cependant, nombreux sont les hommes chez qui le nombre et/ou la mobilité des spermatozoïdes est insuffisante.

L'invention a pour objet une composition médicamenteuse et un médicament destinés à la thérapeutique humaine pour combattre la stérilité masculine en augmentant la mobilité des spermatozoïdes et dans certains cas également leur nombre, en particulier lorsque le nombre des spermatozoïdes est trop faible au départ.

L'invention a également pour objet une composition médicamenteuse et un médicament destinés à la médecine vétérinaire pour le traitement de la stérilité chez les animaux mâles.

Le brevet U.S. 4.540.577 enseigne un complément minéral pour l'alimentation des animaux, comprenant du sulfate de potassium, du sulfate de magnésium, du chlorure de potassium et du soufre, dans la proportion 35,5 : 49,0 : 10 : 5,5, ce qui correspond au rapport en poids S : K : Mg 1 : 3,7 : 1,78.

On a découvert qu'un médicament à base de soufre avait une action bénéfique sur l'augmentation de la mobilité des spermatozoïdes et dans certains cas avait également pour effet d'augmenter le nombre des spermatozoïdes.

Cette action du soufre est optimisée en présence de chlorure de potassium.

L'invention a par conséquent pour objet une composition médicamenteuse et un médicament destinés à la thérapeutique humaine ou animale, comprenant à titre d'élément actif du soufre et du chlorure de potassium.

Le soufre se présente de préférence sous forme de soufre sublimé ou fleur de soufre.

Le rapport en poids soufre:chlorure de potassium est avantageusement compris entre 1 : 0,25 et 1 : 2. Le rapport préféré est de 1 : 1,6.

Selon un mode de réalisation préféré de l'invention, on administre la composition médicamenteuse par voie orale.

On peut également prévoir une administration par voie rectale.

Lorsqu'il est administré par voie orale, le médicament peut comporter un excipient ou véhicule de préférence en poudre ou ne pas comporter d'excipient et être conditionné dans des gélules.

Lorsqu'il comporte des excipients, le médicament peut être conditionné sous forme de pillules, de comprimés, de dragées et sous toute autre forme appropriée.

La composition médicamenteuse et le médicament peuvent contenir en outre tout excipient ou véhicule couramment utilisé pour la préparation des médicaments.

On peut citer notamment :

(a) des véhicules tels que l'amidon, les sucres, le mannitol, le lactose;

(b) des agents liants tels que le carboxycellulose, le carboxyméthylcellulose et autres dérivés cellulosiques, les alginates, la gélatine et la polyvinylpyrrolidone;

(c) des agents humidifiants tels que la glycérine;

2

(d) des lubrifiants tels que le talc;

(e) des parfums et des agents améliorant le goût.

Des formes galéniques particulièrement avantageuses pour la voie orale sont les gélules, les comprimés, les dragées, mais l'on peut administrer la composition médicamenteuse sous toute forme galénique destinée à la voie orale.

Pour les suppositoires à administrer par voie rectale, on peut utiliser des excipients tels que le beurre de cacao, les huiles hydrogénées, les huiles hydrogénées et polyoxyéthylénées, les glycérides semisynthétiques solides, les polyoxyéthylèneglycols ainsi que les autres excipients habituellement employés pour la confection des suppositoires.

La dose journalière recommandée est d'environ 1 g de soufre pour la quantité correspondante de chlorure de potassium.

Pour un rapport soufre : chlorure de potassium de 1 : 1,6, la dose journalière correspond donc à 1 g de soufre et 1,6 g de chlorure de potassium.

Une posologie recommandée consiste à administrer 2 gélules à midi et 2 gélules le soir, à absorber au milieu des repas.

La durée recommandée pour le traitement est de quatre à huit semaines.

Pendant le traitement, il est préférable de ne pas absorber des antibiotiques qui ont une réaction défavorable sur les spermatozoïdes.

Lorsque la composition médicamenteuse ou le médicament est destiné à la médecine vétérinaire, on calcule les doses à administrer à l'animal en multipliant les doses pour les humains par un facteur P/70 où P est égal au poids moyen de l'animal.

Le médicament selon l'invention a été testé sur l'animal et chez des hommes qui étaient volontaires.

Les exemples ci-après illustrent l'invention.

## EXEMPLE 1

Le spermogramme du volontaire n° 1 présente avant le traitement les caractéristiques suivantes.

|  | EXAMEN DANS LA | | | |
|---|---|---|---|---|
|  | 1ère heure | 2ème heure | 4ème heure | 6ème heure |
| Spermatozoïdes |  |  |  |  |
| vivants<br>morts | 60%<br>40% |  |  |  |
| Spermatozoïdes : |  |  |  |  |
| à mobilité normale<br>à mobilité diminuée<br>immobiles | 0%<br>1%<br>99% | 0%<br>0%<br>100% |  |  |

Examen après un traitement de deux mois comportant l'injection de quatre gélules par jour (deux à midi et deux le soir), chaque gélule contenant 0,25 g de soufre et 0,40 g de chlorure de potassium.

| | EXAMEN DANS LA | | | |
|---|---|---|---|---|
| | 1ère heure | 2ème heure | 4ème heure | 6ème heure |
| Spermatozoïdes | | | | |
| vivants | 85% | | | |
| morts | 15% | | | |
| Spermatozoïdes : | | | | |
| à mobilité normale | 74% | 64% | 40% | 38% |
| à mobilité diminuée | 7% | 16% | 14% | 12% |
| immobiles | 19% | 20% | 46% | 50% |

On constate que le traitement a énormément amélioré la mobilité des spermatozoïdes.

EXEMPLE 2

Le spermogramme du volontaire n° 2 comporte les caractéristiques suivantes avant le traitement. Nombre total de spermatozoïdes dans l'éjaculat : 0,9 million.

| | EXAMEN DANS LA | | | |
|---|---|---|---|---|
| | 1ère heure | 2ème heure | 4ème heure | 8ème heure |
| Spermatozoïdes | | | | |
| vivants | 15% | 10% | 5% | 5% |

Après un traitement de quatre semaines dans les mêmes conditions que pour l'exemple 1, le spermogramme présente les caractéristiques suivantes :

| Nombre total des spermatozoïdes dans l'éjaculat : 2,8 millions. | | | | |
|---|---|---|---|---|
| Spermatozoïdes : | | | | |
| à mobilité normale | 60% | 60% | 40% | 30% |

On constate qu'à la suite du traitement, le nombre de spermatozoïdes a été multiplié par trois et la mobilité a beaucoup augmentée et le pourcentage des spermatozoïdes à mobilité normale atteint 60%, ce qui est considéré comme satisfaisant.

EXEMPLE 3

Le spermogramme du volontaire n° 3 présente avant le traitement les caractéristiques suivantes :

| | | EXAMEN | | |
|---|---|---|---|---|
| | | à l'émission | après une heure | après quatre heures |
| Spermatozoïdes | | | | |
| normaux | 80% | | | |
| irréguliers | 20% | | | |
| Spermatozoïdes mobiles | | 42% | 31% | 29% |
| mobiles sur place | | 4% | 6% | 4% |
| immobiles | | 54% | 63% | 67% |

Examen après un traitement de deux mois dans les mêmes conditions que pour l'exemple 1, le spermogramme présentant les caractéristiques suivantes :

|  |  | EXAMEN | | |
|---|---|---|---|---|
| Spermatozoïdes |  | à l'émission | après une heure | après quatre heures |
| normaux | 84% |  |  |  |
| irréguliers | 16% |  |  |  |
| Spermatozoïdes mobiles |  | 77% | 58% | 47% |
| mobiles sur place |  | 6% | 10% | 8% |
| immobiles |  | 17% | 33% | 45% |

On constate que le traitement a presque doublé le nombre de spermatozoïdes mobiles.

EXEMPLE 4

Le spermogramme du volontaire n°4 présente avant le traitement les caractéristiques suivantes : Nombre total de spermatozoïdes dans l'éjaculat :

|  | EXAMEN DANS LA | | | |
|---|---|---|---|---|
| Spermatozoïdes | 1ère heure | 2ème heure | 4ème heure | 6ème heure |
| vivants | 25% |  |  |  |
| morts | 75% |  |  |  |
| Spermatozoïdes : |  |  |  |  |
| à mobilité normale | 0% | 0% | 0% | 0% |

Examen après un traitement de huit semaines dans les mêmes conditions que pour l'exemple 1, le spermogramme présentant les caractéristiques suivantes : Nombre total de spermatozoïdes dans l'éjaculat : 4,2 millions.

|  | EXAMEN DANS LA | | | |
|---|---|---|---|---|
| Spermatozoïdes | 1ère heure | 2ème heure | 4ème heure | 6ème heure |
| vivants | 85% |  |  |  |
| morts | 15% |  |  |  |
| Spermatozoïdes : |  |  |  |  |
| à mobilité normale | 60% | 55% | 40% | 20% |
| à mobilité diminuée | 20% | 20% | 20% | 25% |
| immobiles | 20% | 25% | 40% | 55% |

On constate que le traitement a plus que triplé le nombre de spermatozoïdes vivants, a augmenté le nombre total des spermatozoïdes ainsi que leur mobilité.

**Revendications**

1. Médicament destiné au traitement de la stérilité masculine chez l'homme et l'animal, comprenant à titre d'éléments actifs du soufre et du chlorure de potassium, caractérisé par le fait qu'il ne comprend pas du sulfate double de potassium et de magnésium et par le fait que le rapport en poids soufre:chlorure de potassium est compris entre 1 : 0,25 et 1 : 2.

2. Médicament selon la revendication 1, caractérisé par le fait que le rapport soufre:chlorure de potassium

EP 0 307 616 B1

est de 1 : 1,6.

**3.** Médicament selon la revendication 1 ou 2, caractérisé par le fait qu'il contient un excipient solide et se présente sous forme de comprimés, de pilules, ou sous toute autre forme solide.

**4.** Médicament selon les revendications 1 à 3, caractérisé par le fait qu'il renferme également un adjuvant choisi parmi les véhicules, les agents liants, les agents humidifiants, les lubrifiants, les parfums et les agents améliorant le goût.

**5.** Médicament selon la revendication 1 ou 2, caractérisé par le fait qu'il ne contient pas d'excipient et se présente sous forme de gélules.

**6.** Médicament selon les revendications 1 ou 2, caractérisé par le fait qu'il se présente sous forme de suppositoires.

**7.** Médicament selon les revendications 1 à 6, destiné au traitement de la stérilité masculine due à l'insuffisance du nombre ou de mobilité des spermatozoïdes.

**8.** Produit vétérinaire selon la revendication 1 ou 2, destiné au traitement de la stérilité chez le mâle.

**9.** Produit vétérinaire selon la revendication 8, destiné au traitement de l'insuffisance du nombre ou de mobilité des spermatozoïdes.

**10.** Utilisation d'une composition comprenant du soufre et du chlorure de potassium pour la préparation d'un médicament destiné au traitement de la stérilité masculine chez l'homme.

**11.** Utilisation d'une composition comprenant du soufre et du chlorure de potassium pour la préparation d'un produit vétérinaire destiné au traitement de la stérilité chez le mâle.

**Claims**

**1.** Medicinal product intended for the treatment of male sterility in man and in animals, comprising as active elements, sulphur and potassium chloride, characterised in that it does not contain double sulphate of potassium and of magnesium and in that the sulphur:potassium chloride weight ratio is between 1:0.25 and 1:2.

**2.** Medicinal product according to Claim 1, characterised in that the sulphur:potassium chloride ratio is 1:1.6.

**3.** Medicinal product according to Claim 1 or 2, characterised in that it contains a solid excipient and is provided in the form of tablets, pills or in any other solid form.

**4.** Medicinal product according to Claims 1 to 3, characterised in that it also contains an adjuvant chosen from vehicles, binding agents, moisturising agents, lubricants, perfumes and taste improving agents.

**5.** Medicinal product according to Claim 1 or 2, characterised in that it does not contain an excipient and is provided in the form of hard gelatin capsules.

**6.** Medicinal product according to Claims 1 or 2, characterised in that it is provided in the form of suppositories.

**7.** Medicinal product according to Claims 1 to 6, intended for the treatment of male sterility due to the insufficiency of the number or of the mobility of sperm cells.

**8.** Veterinary product according to Claim 1 or 2, intended for the treatment of sterility in the male organism.

**9.** Veterinary product according to Claim 8, intended for the treatment of the insufficiency of the number

6

or of the mobility of sperm cells.

10. Use of a composition containing sulphur and potassium chloride for the preparation of a medicinal product intended for the treatment of male sterility in man.

11. Use of a composition containing sulphur and potassium chloride for the preparation of a veterinary product intended for the treatment of sterility in the male organism.

**Patentansprüche**

1. Medikament zur Behandlung der männlichen Sterilität bei Mensch und Tier, enthaltend als aktive Bestandteile Schwefel und Kaliumchlorid, dadurch gekennzeichnet, daß es kein doppeltes Sulfat von Kalium und Magnesium enthält und dadurch, daß das Gewichtsverhältnis Schwefel : Kaliumchlorid zwischen 1 : 0,25 und 1 : 2 liegt.

2. Medikament nach Anspruch 1, dadurch gekennzeichnet, daß das Verhältnis Schwefel : Kaliumchlorid 1 : 1,6 ist.

3. Medikament nach Anspruch 1 oder 2, dadurch gekennzeichnet, daß es einen festen Zusatzstoff enthält und sich in Form von Tabletten, Pillen oder unter irgendeiner anderen festen Form darstellt.

4. Medikament nach den Ansprüchen 1 bis 3, dadurch gekennzeichnet, daß es auch einen Zusatzstoff enthält, der unter den Vehikeln , den Bindemitteln, den Befeuchtungsmitteln , den Schmiermitteln, den Parfums und den Mitteln zur Verbesserung des Geschmacks ausgewählt wird.

5. Medikament nach den Ansprüchen 1 oder 2, dadurch gekennzeichnet, daß es keinen Zusatzstoff enthält und sich in Form von Gelatinekapseln darstellt.

6. Medikament nach Anspruch 1 oder 2, dadurch gekennzeichnet, daß es sich in Form von Suppositoren darstellt.

7. Medikament nach Ansprüchen 1 bis 6 zur Behandlung der männlichen Sterilität aufgrund der Insuffizienz der Zahl oder der Mobilität der Spermatozoiden.

8. Veterinäres Produkt nach Anspruch 1 oder 2 zur Behandlung der Sterilität beim Männchen.

9. Veterinäres Produkt nach Anspruch 8 zur Behandlung der Insuffizienz der Zahl oder Mobilität der Spermatozoiden.

10. Verwendung einer Zusammensetzung, die Schwefel und Kaliumchlorid aufweist, zur Herstellung eines Medikamentes zur Behandlung der maskulinen Sterilität beim Mann.

11. Verwendung einer Zusammensetzung, die Schwefel und Kaliumchlorid enthält, zur Herstellung eines veterinären Produktes zur Behandlung der Sterilität beim Männchen.